# DEMANDE DE BREVET EUROPEEN

(11) **EP 1 693 458 A1**
(43) Date de publication de la demande: **23.08.2006**
(21) Numéro de dépôt: 05290363.0
(22) Date de dépôt: 17.02.2005
(51) Int. Cl.: C12N 15/62, A61K 47/48

(54) **Peptides inhibiteurs intracellulaires**

(71) Demandeur: UNIVERSITE PIERRE ET MARIE CURIE, 75005 Paris (FR); CENTRE NATIONAL DE LA RECHERCHE SCIENTIFIQUE (CNRS), 75794 Paris Cedex 16 (FR)
(72) Inventeur: Caboche, Jocelyne, 78490 Vicq (FR); Vanhoutte, Peter, 75013 Paris (FR)
(74) Mandataire: Perin, Georges

(57) **Abrégé**

La présente invention concerne des peptides utiles comme inhibiteurs spécifiques des voies MAP kinases/ERK envers un substrat donné dans un compartiment subcellulaire donné.

SEQUENCE LISTING

<110>  CNRS
       Université Pierre et Marie Curie

<120>  Peptides inhibiteurs

<130>  GBu/CN/VF - EP05290363

<160>  41

<170>  PatentIn version 3.3

<210>  1
<211>  10
<212>  PRT
<213>  artificial

<220>
<223>  artificial sequence

<400>  1

Gly Arg Lys Lys Arg Arg Gln Arg Arg Arg
1               5                   10


<210>  2
<211>  16
<212>  PRT
<213>  artificial

<220>
<223>  artificial sequence

<400>  2

Arg Gln Ile Lys Ile Trp Phe Gln Asn Arg Arg Met Lys Trp Lys Lys
1               5                   10                  15


<210>  3
<211>  7
<212>  PRT
<213>  artificial

<220>
<223>  artificial sequence

<400>  3

Arg Arg Arg Arg Arg Arg Arg
1               5


<210>  4
<211>  9
<212>  PRT
<213>  artificial

<220>

<223>  artificial sequence

<220>
<221>  misc_feature
<222>  (1)..(1)
<223>  Xaa can be any naturally occurring amino acid

<220>
<221>  misc_feature
<222>  (9)..(9)
<223>  Xaa can be any naturally occurring amino acid

<400>  4

Xaa Arg Arg Arg Arg Arg Arg Arg Xaa
1               5

<210>  5
<211>  9
<212>  PRT
<213>  artificial

<220>
<223>  artificial sequence

<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<400>  5

Xaa Arg Arg Arg Xaa Arg Arg Arg Arg
1               5

<210>  6
<211>  9
<212>  PRT
<213>  artificial

<220>
<223>  artificial sequence

<220>
<221>  misc_feature
<222>  (4)..(4)
<223>  Xaa can be any naturally occurring amino acid

<220>
<221>  misc_feature
<222>  (9)..(9)

<223>  Xaa can be any naturally occurring amino acid

<400>  6

Arg Arg Arg Xaa Arg Arg Arg Arg Xaa
1               5


<210>  7
<211>  9
<212>  PRT
<213>  artificial

<220>
<223>  artificial sequence


<220>
<221>  misc_feature
<222>  (8)..(9)
<223>  Xaa can be any naturally occurring amino acid

<400>  7

Arg Arg Arg Arg Arg Arg Arg Xaa Xaa
1               5


<210>  8
<211>  9
<212>  PRT
<213>  artificial

<220>
<223>  artificial sequence


<220>
<221>  misc_feature
<222>  (1)..(2)
<223>  Xaa can be any naturally occurring amino acid

<400>  8

Xaa Xaa Arg Arg Arg Arg Arg Arg Arg
1               5


<210>  9
<211>  11
<212>  PRT
<213>  artificial

<220>
<223>  artificial sequence

<400>  9

Arg Arg Arg Arg Arg Arg Arg Arg Arg Arg Arg
1               5                   10

<210>  10
<211>  13
<212>  PRT
<213>  artificial

<220>
<223>  artificial sequence


<220>
<221>  misc_feature
<222>  (1)..(1)
<223>  Xaa can be any naturally occurring amino acid

<220>
<221>  misc_feature
<222>  (7)..(7)
<223>  Xaa can be any naturally occurring amino acid

<400>  10

Xaa Arg Arg Arg Arg Arg Xaa Arg Arg Arg Arg Arg Arg
1               5                   10


<210>  11
<211>  13
<212>  PRT
<213>  artificial

<220>
<223>  artificial sequence


<220>
<221>  misc_feature
<222>  (6)..(6)
<223>  Xaa can be any naturally occurring amino acid

<220>
<221>  misc_feature
<222>  (13)..(13)
<223>  Xaa can be any naturally occurring amino acid

<400>  11

Arg Arg Arg Arg Arg Xaa Arg Arg Arg Arg Arg Arg Xaa
1               5                   10


<210>  12
<211>  7
<212>  PRT
<213>  artificial

<220>
<223>  artificial sequence

<400>  12

Pro Lys Lys Lys Arg Lys Val
1               5

<210>  13
<211>  16
<212>  PRT
<213>  artificial

<220>
<223>  artificial sequence

<400>  13

Lys Arg Pro Ala Ala Ile Lys Lys Ala Gly Gln Ala Lys Lys Lys Lys
1               5                   10                  15

<210>  14
<211>  15
<212>  PRT
<213>  artificial

<220>
<223>  artificial sequence

<400>  14

Arg Gln Ala Arg Arg Asn Arg Arg Asn Arg Arg Arg Arg Trp Arg
1               5                   10                  15

<210>  15
<211>  12
<212>  PRT
<213>  artificial

<220>
<223>  artificial sequence

<220>
<221>  misc_feature
<222>  (1)..(1)
<223>  Xaa can be any naturally occurring amino acid

<220>
<221>  misc_feature
<222>  (3)..(5)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(12)
<223>  Xaa can be any naturally occurring amino acid

<400>  15

Xaa Leu Xaa Xaa Xaa Leu Xaa Xaa Leu Xaa Leu Xaa
1               5                   10


<210>  16
<211>  12
<212>  PRT
<213>  artificial

<220>
<223>  artificial sequence


<220>
<221>  misc_feature
<222>  (1)..(1)
<223>  Xaa can be any naturally occurring amino acid

<220>
<221>  misc_feature
<222>  (3)..(5)
<223>  Xaa can be any naturally occurring amino acid

<220>
<221>  misc_feature
<222>  (7)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(12)
<223>  Xaa can be any naturally occurring amino acid

<400>  16

Xaa Leu Xaa Xaa Xaa Leu Xaa Xaa Leu Xaa Arg Xaa
1               5                   10


<210>  17
<211>  12
<212>  PRT
<213>  artificial

<220>
<223>  artificial sequence

<400>  17

Ala Leu Gln Lys Lys Leu Glu Glu Leu Glu Leu Asp
1               5                   10

<210>  18
<211>  12
<212>  PRT
<213>  artificial

<220>
<223>  artificial sequence

<400>  18

Thr Leu Trp Gln Phe Leu Leu Gln Leu Leu Leu Asp
1               5                   10

<210>  19
<211>  12
<212>  PRT
<213>  artificial

<220>
<223>  artificial sequence

<400>  19

Thr Leu Trp Gln Phe Leu Leu Gln Leu Leu Arg Glu
1               5                   10

<210>  20
<211>  18
<212>  PRT
<213>  artificial

<220>
<223>  artificial sequence

<400>  20

Ser Pro Ala Lys Leu Ser Phe Gln Phe Pro Ser Gly Ser Ala Gln Val
1               5                   10                  15

His Ile

<210>  21
<211>  19
<212>  PRT
<213>  artificial

<220>
<223>  artificial sequence

<400>  21

Ser Pro Ala Arg Leu Gln Gly Ala Asn Thr Leu Phe Gln Phe Pro Ser

1               5                   10                  15

Val Leu Asn

<210>  22
<211>  19
<212>  PRT
<213>  artificial

<220>
<223>  artificial sequence

<400>  22

Ser Pro Ala Arg Leu Gln Gly Pro Ser Thr Leu Phe Gln Phe Pro Thr
1               5                   10                  15

Leu Leu Asn

<210>  23
<211>  18
<212>  PRT
<213>  artificial

<220>
<223>  artificial sequence

<400>  23

Met Ala Val Leu Asp Arg Gly Thr Ser Thr Thr Thr Val Phe Asn Phe
1               5                   10                  15

Pro Val

<210>  24
<211>  16
<212>  PRT
<213>  artificial

<220>
<223>  artificial sequence

<400>  24

Pro Asn Pro Ser Pro Gly Gln Arg Asp Ser Arg Phe Ser Phe Pro Asp
1               5                   10                  15

<210>  25
<211>  18
<212>  PRT
<213>  artificial

<220>
<223>  artificial sequence

<400>  25

Ser Leu Thr Pro Thr Ala Ala His Ser Gly Ser His Leu Phe Gly Phe
1               5                   10                  15

Pro Pro

<210>  26
<211>  17
<212>  PRT
<213>  artificial

<220>
<223>  artificial sequence

<400>  26

Lys Gly Arg Lys Pro Arg Asp Leu Glu Leu Pro Leu Ser Pro Ser Leu
1               5                   10                  15

Leu

<210>  27
<211>  16
<212>  PRT
<213>  artificial

<220>
<223>  artificial sequence

<400>  27

Arg Ser Lys Lys Pro Lys Gly Leu Gly Leu Ala Pro Thr Leu Val Ile
1               5                   10                  15

<210>  28
<211>  18
<212>  PRT
<213>  artificial

<220>
<223>  artificial sequence

<400>  28

Lys Ala Lys Lys Pro Lys Gly Leu Glu Ile Ser Ala Pro Pro Leu Leu
1               5                   10                  15

Val Leu

<210>  29
<211>  17
<212>  PRT
<213>  artificial

<220>
<223>  artificial sequence

<400>  29

Ser Ser Ile Leu Ala Gln Arg Arg Val Arg Lys Leu Pro Ser Thr Thr
1               5                   10                  15

Leu

<210>  30
<211>  19
<212>  PRT
<213>  artificial

<220>
<223>  artificial sequence

<400>  30

Arg Arg Ser Thr Leu Ala Gln Arg Arg Gly Ile Lys Lys Ile Thr Ser
1               5                   10                  15

Thr Ala Leu

<210>  31
<211>  18
<212>  PRT
<213>  artificial

<220>
<223>  artificial sequence

<400>  31

Ser Ser Asn Leu Ala Gln Arg Arg Gly Met Lys Arg Leu Thr Ser Thr
1               5                   10                  15

Arg Leu

<210>  32
<211>  18
<212>  PRT
<213>  artificial

<220>
<223>  artificial sequence

<400>  32

Lys Ser Arg Leu Ala Arg Arg Arg Ala Leu Ala Gln Ala Gly Arg Ser
1               5                   10                  15

Arg Asp

<210>  33
<211>  19
<212>  PRT
<213>  artificial

<220>
<223>  artificial sequence

<400>  33

Gln Ser Lys Leu Ala Gln Arg Arg Gln Arg Ala Ser Leu Ser Ser Ala
1               5                   10                  15

Thr Pro Val

<210>  34
<211>  18
<212>  PRT
<213>  artificial

<220>
<223>  artificial sequence

<400>  34

Lys Ala Pro Leu Ala Lys Arg Arg Lys Met Lys Lys Thr Ser Thr Ser
1               5                   10                  15

Thr Glu

<210>  35
<211>  17
<212>  PRT
<213>  artificial

<220>
<223>  artificial sequence

<400>  35

Arg Phe Ser Thr Ile Val Arg Arg Arg Ala Lys Gly Ala Lys Gly Ala

1               5                   10                  15

Gly

<210>  36
<211>  18
<212>  PRT
<213>  artificial

<220>
<223>  artificial sequence

<400>  36

Pro Gly Ile Met Leu Arg Arg Leu Gln Lys Gly Asn Leu Pro Val Arg
1               5                   10                  15

Ala Leu

<210>  37
<211>  17
<212>  PRT
<213>  artificial

<220>
<223>  artificial sequence

<400>  37

Leu Pro Ala Leu Leu Leu Arg Arg Leu Arg Arg Gly Ser Leu Ser Val
1               5                   10                  15

Arg

<210>  38
<211>  16
<212>  PRT
<213>  artificial

<220>
<223>  artificial sequence

<400>  38

Gly Leu Gln Glu Arg Arg Gly Ser Asn Val Ser Leu Thr Leu Asp Met
1               5                   10                  15

<210>  39
<211>  42
<212>  PRT
<213>  artificial

<220>
<223>  artificial sequence

<400>  39

Gly Arg Lys Lys Arg Arg Gln Arg Arg Arg Cys Cys Thr Leu Trp Gln
1               5                   10                  15

Phe Leu Leu His Leu Leu Leu Asp Ser Pro Ala Lys Leu Ser Phe Gln
            20                  25                  30

Phe Pro Ser Gly Ser Ala Gln Val His Ile
        35                  40

<210>  40
<211>  30
<212>  PRT
<213>  artificial

<220>
<223>  artificial sequence

<400>  40

Gly Arg Lys Lys Arg Arg Gln Arg Arg Arg Pro Pro Ser Pro Ala Lys
1               5                   10                  15

Leu Ser Phe Gln Phe Pro Ser Gly Ser Ala Gln Val His Ile
            20                  25                  30

<210>  41
<211>  42
<212>  PRT
<213>  artificial

<220>
<223>  artificial sequence

<400>  41

Gly Arg Lys Lys Arg Arg Gln Arg Arg Arg Cys Cys Thr Leu Trp Gln
1               5                   10                  15

Phe Leu Leu His Leu Leu Leu Asp Pro Gly Ile Met Leu Arg Arg Leu
            20                  25                  30

Gln Lys Gly Asn Leu Pro Val Arg Ala Leu
        35                  40

## Description

La présente invention concerne des peptides permettant l'inhibition sélective de la voie des MAP kinases de type ERK envers un substrat donné et dans un compartiment cellulaire donné.

Les MAP kinases (Mitogen Activated Protein kinases) sont des protéines ubiquitaires impliquées dans des fonctions cellulaires variées. Ces protéines assurent la transduction de signaux intracellulaires : de la surface de la cellule, vers le noyau. Trois grandes familles de MAP kinases (ERK, p38, JNK) ont été identifiées, qui correspondent à des voies de signalisation en cascade. Ces voies de signalisation jouent des rôles importants dans les fonctions cellulaires : de l'apoptose à la prolifération, différenciation, voire même la plasticité neuronale Ces fonctions dépendent de façon étroite du type de MAP kinase, d'une part, et pour chaque type de MAP kinase, de sa localisation cellulaire.

Afin d'élucider les mécanismes moléculaires gouvernés par les voies de signalisation ERK ainsi que d'être en mesure d'interférer avec cette cascade de signalisation à un niveau donné, il est utile de disposer d'inhibiteurs spécifiques.

Les composés actuellement disponibles : le PD98059 (2'-amino-3'-méthoxyflavone, un inhibiteur polycyclique azoté) et le U0126 (1,4-Diamino-2,3-dicyano-1,4-bis(2-aminophenylthio)butadiène) sont des inhibiteurs spécifiques des MEKs, les kinases en amont des ERKs. Toutefois, leur action se situe en amont des ERKs, ce qui résulte en une inhibition complète de leur activation, et par conséquent de tous les substrats en aval, sans discrimination entre ceux-ci et sans distinction de leur localisation cellulaire.

Il serait donc utile de disposer d'inhibiteurs hautement sélectifs des ERKs agissant en aval, sur des substrats spécifiques soit cytoplasmiques, soit nucléaires, et ce afin de minimiser, de préférence totalement éviter, tout effet connexe, voire pléiotrope.

La présente invention fournit des peptides utiles comme inhibiteurs hautement sélectifs des MAP kinases de type ERK vis à vis de leurs substrats nucléaires ou cytoplasmiques.

Selon la présente invention, 'MAP kinase de type ERK' ou ERK désigne toute MAP kinase ERK. En particulier, ladite MAP kinase de type ERK peut être une MAP kinase mammifère, en particulier humaine, primate ou murine. Elle peut aussi être non mammifère (lamproie, zebrafish, C. Elegans, drosophile, xénope)

Selon la présente invention, 'inhibiteur de ERKs' ou 'inhibiteur de MAP kinase de type ERK' désigne tout composé permettant d'inhiber la fonction kinase de ERK sur au moins un substrat donné.

Selon la présente invention, 'peptide' ou 'chaîne peptidique' désigne toute chaîne d'acides aminés. Ladite chaîne d'acides aminés comporte en général de 2 à 100 résidus, préférentiellement de 5 à 75 résidus, plus préférentiellement de 10 à 50 résidus. (Voir définition IUPAC,
http://www.chem.qmul.ac.uk/iupac/AminoAcid/A 1113 .html#AA11).

De préférence, ladite chaîne contient 2, 3, 4, 5, 6, 7, 8, 9, 10, 11, 12, 13, 14, 15, 16, 17, 18, 19, 20, ..., 50, ..., 100 résidus acides aminés.

Selon la présente invention, le terme 'acide aminé' ou 'résidu acide aminé' désigne tout résidu acide aminé connu de l'homme du métier (Voir par exemple : N. Sewald, H.-D. Jakubke, Peptides: Chemistry and Biology 2002, Wiley-VCH Verlag GmbH, Weinheim; nomenclature IUPAC
http://www.chem.qmul.ac.uk/iupac/AminoAcid/).

Ceci comprend les acides aminés naturels (incluant, par exemple, selon le code à trois lettres, Ala, bAla, Arg, Asn, Asp, Cys, Gln, Glu, Gly, His, Ile, Leu, Lys, Met, Phe, Pro, Ser, Thr, Trp, Tyr, Val), ainsi que des acides aminés rares et/ou synthétiques et leurs dérivés (incluant par exemple Aad, Abu, Acp, Ahe, Aib, Apm, Dbu, Des, Dpm, Hyl, MeLys, MeVal, Nva, HAO, NCap, Abu, Aib, MeXaa et similaires (Voir par exemple : J. S. Nowick, J. O. Brower, J. Am. Chem. Soc. 2003, 125, 876-877; R. Aurora, G. D. Rose, Protein Science 1998, 7, 21-38; W. Maison, E. Arce, P. Renold, R. J. Kennedy, D. S. Kemp, J. Am. Chem. Soc. 2001, 123, 10245-10254; D. Obrecht, M. Altorfer, J. A. Robinson, Adv. Med. Chem. 1999, 4, 1-68; K. Müller, D. Obrecht, A. Knierzinger, C.

Stankovic, C. Spiegler, W. Bannwarth, A. Trzeciak, G. Englert, A. M. Labhard, P. Schönholzer in Perspectives in Medicinal Chemistry, (Eds.: B. Testa, E. Kyburz, W. Fuhrer, R. Giger), Verlag Helv. Chim. Acta, Basel, 1993, pp. 513-533; F. Formaggio, A. Bettio, V. Moretto, M. Crisma, C. Toniolo, Q. B. Broxterman, J. Peptide Sci. 2003, 9, 461-466).

Ledit résidu acide aminé ou son dérivé peut être tout isomère de celui-ci, en particulier tout isomère chiral, par exemple l'isoforme L- ou D-, et leurs mélanges. L'isoforme D- présente l'avantage d'une meilleure stabilité.

Par 'dérivé d'acide aminé', on désigne ici tout dérivé d'acide aminé, en particulier tout dérivé connu de l'homme du métier. (Voir par exemple : N. Sewald, H.-D. Jakubke, Peptides: Chemistry and Biology 2002, Wiley-VCH Verlag GmbH, Weinheim; nomenclature IUPAC
http://www.chem.qmul.ac.uk/iupac/AminoAcid/).

Par exemple, les dérivés d'acides aminés incluent des résidus dérivables d'acides aminés naturels portant des chaînes latérales supplémentaires, par exemple des chaînes latérales alkyl, et/ou des substitutions d'hétéroatomes.

La notion de 'séquence d'acides aminés est connue de l'homme du métier. Une séquence d'acides aminés comprend au moins deux résidus liés de manière covalente par au moins une liaison peptidique.

Par la suite, les séquences d'acides aminés seront données en utilisant le code à une lettre.

Ledit peptide peut être obtenu par des procédés connus de l'homme du métier, par exemple ledit peptide peut être obtenu par des méthode de synthèse, telle que la synthèse sur support solide ou la synthèse en solution (peptides de synthèse), ou des techniques issues de la biologie moléculaire (peptide recombinant).

La présente invention concerne un peptide comprenant:
- Au moins une séquence d'acides aminés permettant la pénétration dudit peptide dans une cellule ;
- Une séquence d'acides aminés d'adressage intracellulaire choisie parmi les NES ;
- Optionnellement une séquence d'adressage intracellulaire choisie parmi les NLS ;
- Une séquence d'acides aminés correspondant à une séquence d'ancrage (« docking domain ») d'un substrat d'une MAP kinase de type ERK ;
- Optionnellement, au moins une séquence « espaceur » (spacer) ;
- Optionnellement une séquence de clivage enzymatique entourée ou non par des séquences espaceur.

Par 'séquence d'acides aminés permettant la pénétration dudit peptide dans une cellule', on désigne selon la présente invention toute séquence d'acides aminés facilitant et/ou médiant le transport dudit peptide de l'extérieur d'une cellule vers son intérieur. De telles séquences sont connues de l'homme du métier. Ladite séquence permettant la pénétration dudit peptide dans une cellule, peut être choisie selon le type cellulaire de ladite cellule, afin d'optimiser l'efficacité de pénétration.

Selon un mode de réalisation, ladite séquence permettant la pénétration dudit peptide dans une cellule a une longueur de 2 à 20 résidus, notamment 6, 7, 8, 9, ..., 17, 18, 19 ou 20 résidus.

Selon un mode de réalisation, ladite séquence permettant la pénétration dudit peptide dans une cellule est choisie parmi : la séquence du peptide pénétrant de HIV-TAT, la Pénétratine, une séquence de sept à onze arginine, une séquence dite « séquence X7/11R ».

Par « séquence X7/11R », on entend toute séquence peptidique de 7 à 20 acides aminés contenant entre sept et onze résidus arginine (7/11R), dans laquelle les résidus arginine (R) peuvent être placés de façon aléatoire au sein de ladite séquence. Des exemples sont donnés plus bas, mais l'homme du métier est en mesure de décliner les autres possibilités.

Selon un mode de réalisation, ladite séquence permettant la pénétration dudit peptide dans une cellule est choisie parmi :

| SEQ ID No : | Séquence -> pénétration | Origine |
|---|---|---|
| 1 | GRKKRRQRRR | HIV-TAT |
| 2 | RQIKIWFQNRRMKWKK | Pénétratine |
| 3 | RRRRRRR | 7R |
| 4 | XRRRRRRRX | X7R (exemple) |
| 5 | XRRRXRRRR | X7R (autre exemple) |
| 6 | RRRXRRRRX | X7R (autre exemple) |
| 7 | RRRRRRRXX | X7R (autre exemple) |
| 8 | XXRRRRRRR | X7R (autre exemple) |
| 9 | RRRRRRRRRRR | 11R |
| 10 | XRRRRRXRRRRRR | X11R (autre exemple) |
| 11 | RRRRRXRRRRRRX | X11R (autre exemple) |

La notion de NLS (« Nuclear Localizing Signal », signal de localisation nucléaire) est connue de l'homme du métier. Il s'agit généralement d'une séquence d'acides aminés permettant l'adressage d'une protéine donnée au noyau, via le phénomène d'import nucléaire.

Selon un mode de réalisation, ladite séquence NLS est une séquence riche en acides aminés basiques (Arginine ou Lysine).

Selon un mode de réalisation, ladite séquence NLS a une longueur de 2 à 20 résidus, notamment 6, 7, 8, 9, ..., 17, 18, 19 ou 20 résidus.

Selon un mode de réalisation, ladite séquence NLS est choisie parmi :

| SEQ ID No : | Séquence NLS | origine |
|---|---|---|
| 12 | P**KKKRK**V | SV40 large T-antigen |
| 13 | **KR**PAAI**KK**AGQA**KKKK** | nucléoplasmine |
| 14 | **R**QA**RR**N**RR**N**RRRR**W**R** | HIV1Rev |
| 1 | GRKKRRQRRR | HIV-TAT |
| 2 | RQIKIWFQNRRMKWKK | Pénétratine |
| 3 | RRRRRRR | 7R |
| 9 | RRRRRRRRRRR | 11R |

La notion de NES (« Nuclear Export Signal », signal d'export nucléaire) est connue de l'homme du métier. Ce sont généralement des séquences d'acides aminés médiant l'export nucléaire, résultant en une translocation d'une protéine donnée du noyau vers le cytoplasme.

Selon un mode de réalisation, ladite séquence NES a une longueur de 2 à 20 résidus, notamment 6, 7, 8, 9, ..., 10, 11, 12, ..., 17, 18, 19 ou 20 résidus.

Selon un mode de réalisation, ladite séquence NES est choisie parmi :

| SEQ ID No : | Séquence NES | Origine |
|---|---|---|
| 15 | XLXXXLXXLXLX | Consensus de type Elk-1 |
| 16 | XLXXXLXXLXRX | Consensus de type Net |
| 17 | A**L**QKK**L**EE**L**E**L**D | MAPKK |
| 18 | T**L**WQF**L**LQ**L**L**L**D | Net (substrat des ERK) |
| 19 | T**L**WQF**L**LQ**L**LRE | Elk-1 (substrat des ERK) |

Ladite séquence d'acides aminés correspondant à une séquence d'ancrage d'un substrat d'une MAP kinase de type ERK peut comprendre tout « docking domain » d'un substrat de ERK connu de l'homme du métier.

La notion de « docking domain » est connue de l'homme du métier. Il s'agit généralement d'une portion du substrat d'une MAP kinase qui conditionne de manière spécifique l'interaction et/ou le recrutement entre ledit substrat et ladite MAP kinase. Il s'agit de tout ou une partie d'un site de liaison (« docking site ») dudit substrat pour ladite MAP kinase. La séquence dudit « docking domain » est donc spécifique et sélective pour une interaction donnée.

Ainsi, avantageusement selon l'invention, chacune de ces séquences d'ancrage correspond à une portion (séquences d'acides aminés) d'un substrat des MAP kinases ERKs, portion qui conditionne de manière spécifique l'interaction et/ou le recrutement entre ledit substrat et ladite MAP kinase de type ERK.

Selon un mode de réalisation de la présente invention, ladite séquence d'acides aminés correspondant à une séquence d'ancrage d'un substrat d'une MAP kinase de type ERK, a une longueur de 12-25 résidus, de préférence 13, 14, 15, 16, 17, 18, 19, 20 ,21, 22, 23 ou 24 résidus.

Selon un mode de réalisation, ladite séquence « docking domain» est choisie parmi les séquences « docking domain de type FXFP » et « docking domain de type D ».

Selon un mode de réalisation, ladite séquence « docking domain» a une longueur de 12-25 résidus, de préférence 13, 14, 15, 16, 17, 18, 19, 20 ,21, 22, 23 ou 24 résidus.

Par séquence « docking domain de type D », on entend ici toute séquence « docking domain » de type D connue de l'homme du métier. Ceci inclut notamment les séquences d'acides aminés hydrophobes suivie d'une séquence d'amino acides basiques ; les séquences d'acides aminés hydrophobes suivi d'une séquence d'amino acides basiques et d'une séquence 'leucine X leucine' (LXL) ; les séquences d'acides aminés basiques suivi de la séquence 'leucine X leucine' puis d'une séquence d'amino acides hydrophobes.

Par séquence « docking domain de type FXFP », on entend ici toutes séquences « docking domain » de type FXFP connues de l'homme du métier. Ceci inclut notamment les séquences des domaines dits « FXFP » et les séquences Nter et

Cter flanquantes correspondantes dudit substrat).

Selon un mode de réalisation, ladite séquence « docking domain » est choisie parmi :

| SEQ ID No : | Séquence « docking domain » | Type du docking domain | Substrat de ERK |
|---|---|---|---|
| 20 | SPAKLS**FQFP**SGSAQVHI | FXFP | Elk-1 |
| 21 | SPARLQGANTL**FQFP**SVLN | FXFP | Sap-1 |
| 22 | SPARLQGPSTL**FQFP**TLLN | FXFP | Sap-2 |
| 23 | MAVLDRGTSTTTV**FNFP**V | FXFP | MKP-1 |
| 24 | PNPSPGQRDSR**FSFP**D | FXFP | KSR |
| 25 | SLTPTAAHSGSHL**FGFP**P | FXFP | GATA-2 |
| 26 | KGRKPRDLELPLSPSLL | D | Elk-1 |
| 27 | RSKKPKGLGLAPTLVI | D | Sap-1 |
| 28 | KAKKPKGLEISAPPLLVL | D | Sap-2 |
| 29 | SSILAQRRVRKLPSTTL | D | Rsk-1 |
| 30 | RRSTLAQRRGIKKITSTAL | D | Rsk-2 |
| 31 | SSNLAQRRGMKRLTSTRL | D | Rsk-3 |
| 32 | KSRLARRRALAQAGRSRD | D | Mnk-1 |
| 33 | QSKLAQRRQRASLSSATPV | D | Mnk-2 |
| 34 | KAPLAKRRKMKKTSTSTE | D | Msk-1 |
| 35 | RFSTIVRRRAKGAKGAG | D | MKP-1 |
| 36 | PGIMLRRLQKGNLPVRAL | D | MKP-3 |
| 37 | LPALLLRRLRRGSLSVR | D | MKP-4 |
| 38 | GLQERRGSNVSLTLDM | D | STEP |

Un même substrat peut parfois comporter plusieurs « docking domains » pour ERK. C'est le cas par exemple de Elk-1 et MKP-1 vis-à-vis de ERK. Dans ce cas l'une ou l'autre des séquences « docking domain » pourra être utilisée dans un peptide selon l'invention pour bloquer l'interaction ERK/Substrat. Alternativement l'utilisation conjointe de deux peptides selon l'invention, l'un des peptides contenant une séquence « docking domain », par exemple une séquence FXFP ; et l'autre peptide contenant une autre séquence « docking domain », par exemple une séquence D, permettra d'améliorer l'inhibition à des concentrations subliminaires.

Avantageusement selon l'invention, ledit peptide possède les propriétés suivantes : une fois mis en contact avec une cellule, grâce à ladite séquence d'acides aminés permettant la pénétration dudit peptide dans une cellule, le peptide selon l'invention entre dans ladite cellule. Par la suite, selon la nature de ladite séquence d'adressage intracellulaire, ledit peptide se localise soit dans le noyau (si NLS) soit dans le cytoplasme (si NES). Alternativement, en l'absence de séquence d'adressage intracellulaire supplémentaire, compte tenu de la teneur riche en acides aminés basiques de ladite séquence permettant la pénétration, cette dernière joue également le rôle de NLS, de sorte ledit peptide est localisé dans le noyau. Ainsi, selon la structure dudit peptide selon l'invention, celui-ci adopte avantageusement une localisation intracellulaire spécifique. Ladite séquence « docking domain » joue alors un rôle inhibiteur : elle permet avantageusement de mimer la présence dudit substrat vis-à-vis de la MAP kinase de type ERK, résultant ainsi en une inhibition sélective et spécifique de l'interaction entre ERK et ledit substrat, et ceci, avec une localisation intracellulaire spécifique : selon les cas, l'inhibition est spécifique de l'interaction nucléaire, ou spécifique de l'interaction cytoplasmique, entre ERK et ledit substrat. Ainsi, avantageusement selon l'invention, l'inhibition résultante est spécifique non seulement du couple Substrat/ERK (du fait du « docking domain »), mais encore spécifique sur le plan de la localisation intracellulaire (inhibition sélective de l'interaction nucléaire, ou inhibition sélective de l'interaction cytoplasmique : inhibition différentielle spécifique).

Ladite cellule est une cellule eucaryote, de préférence une cellule d'eucaryote supérieur, par exemple une cellule de mammifère ou une cellule humaine. Ce peut être une cellule en mitose ou une cellule quiescente (post-mitotique), par exemple une cellule neuronale.

Avantageusement selon l'invention, ladite séquence « spacer » optionnelle permet d'assurer une certaine flexibilité conformationnelle entre ladite séquence permettant la pénétration dudit peptide dans une cellule, et ladite séquence « docking domain ». Par exemple, ladite séquence « spacer » peut comprendre au moins un, de préférence plusieurs résidus proline, par exemple 2, 3, ou 4 résidus proline.

Par ailleurs, selon un mode de réalisation, ledit peptide peut comprendre un site de clivage enzymatique, permettant de séparer ladite séquence d'acides aminés de pénétration dudit peptide dans une cellule, du reste dudit peptide. Avantageusement selon l'invention, ledit peptide peut ainsi comprendre deux résidus cystéine consécutifs, permettant ainsi un clivage intracellulaire par la glutathion cytoplasmique (les ponts disulfures existant entre ces deux résidus sont coupés après pénétration dans la cellule). Tout autre site de clivage enzymatique, notamment par une protéase intracellulaire connu de l'homme de métier peut également être utilisé. Selon un mode de réalisation de l'invention, ledit site de clivage peut être un site de clivage par des cystéines-protéases de type caspase ou par la NSE (énolase spécifique des neurones).

Selon un mode de réalisation, le peptide selon l'invention est couplé à au moins un fluorophore, de préférence de manière covalente. Ledit fluorophore peut être tout fluorophore connu de l'homme du métier. En particulier, ledit fluorophore peut être choisi parmi Fam, Hex, Tet, Joe, Rox, Tamra, Max, Edans, colorants Cy tels que Cy5 ou Cy3, Fluorescein, Coumarin, Eosine, Rhodamine, Bodipy, Alexa, Cascade Blue, Yakima Yellow, Lucifer Yellow and Texas Red (marques déposées). Alternativement, ledit peptide peut être biotynilé et visualisé, indirectement, avec de l'avidine marquée avec les fluorophores décrits ci-dessus. Ledit peptide pourra également être couplé à un marqueur enzymatique, par exemple de type beta-galactosidase. Avantageusement selon l'invention lesdits fluorophores, biotine ou enzyme (beta-galactosidase par exemple) sont localisés en région C-terminale ou N-terminale du site d'ancrage dudit peptide de façon à pouvoir le localiser chez l'animal entier *in vivo,* sur une préparation de cellules, *in vitro,* aussi bien que sur une préparation de cellules fixées.

La présente invention concerne également un acide nucléique codant un peptide tel que décrit précédemment. Pour un peptide donné, l'homme du métier saura identifier quelle(s) séquence(s) d'acide nucléique code(nt) pour un tel peptide, en se basant sur le code génétique, ses dégénérescences, et l'adaptation des codons selon les espèces.

La présente invention concerne en outre un vecteur d'expression comprenant un acide nucléique codant un peptide tel que décrit ci-dessus.

Selon un mode de réalisation, ledit vecteur d'expression est un vecteur d'expression eucaryote.

Ledit vecteur d'expression sera avantageusement adapté à un type cellulaire donné, selon l'utilisation à laquelle on destine le peptide selon l'invention. Ainsi, l'homme du métier saura concevoir un tel vecteur. Notamment, l'homme du métier saura choisir entre un promoteur constitutif ou tissu-spécifique, permettant l'expression dudit peptide à partir dudit vecteur. En outre, ledit promoteur d'expression peut être choisi parmi les promoteurs constitutifs, les promoteurs inductibles, les promoteurs spécifiques, par exemple tissus-spécifiques.

Selon un mode de réalisation, ledit vecteur d'expression contient un promoteur de type nestine, afin de permettre l'expression précoce dudit peptide au cours du développement.

Selon un autre mode de réalisation, ledit vecteur d'expression comprend au moins un promoteur tissu-spécifique, afin de permettre l'expression dudit peptide dans des tissus ciblés.

De plus, pour un tissu donné, l'expression dudit peptide pourra être restreinte à certaines régions dudit tissu, par exemple, certaine régions du cerveau :
Selon un mode de réalisation, ledit vecteur d'expression contient un promoteur de type CaMKII, afin d'obtenir une expression préférentielle dans l'hippocampe (siège de la mémoire spatiale).
Selon un mode de réalisation, ledit vecteur d'expression contient un promoteur des récepteurs dopaminergiques de type Dl, afin d'obtenir une expression striatum spécifique (siège des processus addictifs).
Selon un mode de réalisation, ledit vecteur d'expression contient un promoteur Tyrosine Hydroxylase pour une expression dans la substance noire compacte (siège des processus dégénératifs dans la maladie de parkinson).
Selon un mode de réalisation, ledit vecteur d'expression contient en outre un promoteur inductible, par exemple un promoteur induit ou réprimé par la tétracycline (système TetOn, TetOff) .
Ledit vecteur d'expression peut contenir une origine de réplication bactérienne permettant sa réplication dans des cellules hôtes bactériennes, typiquement E. coli.
Selon un mode de réalisation, ledit vecteur d'expression est conçu de manière à pouvoir être utilisé pour générer des animaux transgéniques, par exemple des murins transgéniques, qui exprimeront ledit peptide à des moments voulus dans un tissu donné, et au sein dudit tissu (par exemple dans le cerveau), dans une région donnée.
Selon un mode de réalisation, ledit vecteur d'expression est un vecteur viral. Ledit vecteur viral peut être choisi dans le groupe des vecteurs rétroviraux, vecteur viraux canins, vecteurs lentiviraux. Ledit vecteur viral permet alors une expression tissu spécifique : un vecteur rétroviral permet de cibler préférentiellement les cellules en division ; un virus canin permet de cibler des cellules post-mitotiques de type neuronal ; un vecteur lentiviral peut s'intégrer au génome de la cellule hôte sans discrimination. Ledit vecteur viral peut également être utilisé dans le cadre d'une thérapie génique.

La présente invention concerne également un kit contenant au moins un peptide tel que décrit précédemment et/ou au moins un vecteur ou acide nucléique codant pour des peptides tels que décrits ci-dessus. En outre, ledit kit peut contenir des témoins (positifs ou négatifs) sous forme de peptides ou de vecteurs, permettant de conduire des expériences témoins en parallèle des expériences impliquant au moins un peptide selon la présente invention. Par exemple, un peptide témoin négatif peut contenir une séquence 'brouillée' d'acides aminés. Ledit kit peut par ailleurs contenir une notice d'explication.

Selon un mode de réalisation, ledit kit peut comporter au moins deux peptides différents selon l'invention. Lesdits peptides peuvent être destinés à inhiber l'interaction d'une MAP kinase de type ERK avec au moins deux substrats distincts, ou un seul et unique substrat. En effet, un même substrat peut parfois comporter plusieurs « docking domains » pour une même MAP kinase. C'est le cas par exemple de Elk-1 et MKP-1 vis-à-vis de ERK. Dans ce cas l'une ou l'autre des séquences « docking domain » pourra être utilisée dans un peptide selon l'invention pour bloquer l'interaction ERK/Substrat. Alternativement l'utilisation conjointe de deux peptides selon l'invention, l'un des peptides contenant une séquence « docking domain », par exemple une séquence FXFP ; et l'autre peptide contenant une autre séquence « docking domain », par exemple une séquence D, permettra d'améliorer l'inhibition à des concentrations subliminaires.

La présente invention concerne également l'utilisation d'un peptide tel que décrit précédemment comme inhibiteur in vitro ou in vivo de l'activité de ladite MAP kinase de type ERK envers un substrat donné. Ce type d'utilisation couvre des domaines très variés, selon la nature du (des) substrat(s) de ladite MAP kinase de type ERK, le type cellulaire considéré, et le type de stimulation extracellulaire considérée.

Avantageusement, ledit peptide pourra être marqué, par exemple couplé à un marqueur (par exemple, fluorophore, biotine ou beta-galactosidase), et pourra ainsi être testé in vivo chez l'animal entier après injection par voie systémique ou intra-tissulaire. Après injection systémique, ledit peptide pourra être localisé dans les différents tissus, y compris dans le système nerveux central (la présence de ladite séquence permettant la pénétration, permettant le passage de la barrière hémato-encéphalique) grâce au marqueur couplé au peptide.

Ainsi, le peptide selon l'invention est utile dans l'étude de différents types de phénomènes, notamment en neurobiologie (étude du développement, de la plasticité neuronale, des processus addictifs) et cancérologie (régulation du cycle cellulaire).

| Stimulation extracellulaire | Type cellulaire | Substrat de ERK | inhibition | phénomène |
|---|---|---|---|---|
| Neuro-transmetteur | Neurone | Elk-1 MKP-1 | Nucléaire | Toxicomanie Mémoire Apprentissage Survie Plasticité |
| Neuro-transmetteur | Neurone | MKP-3 | Cytoplasmique | Neuro-dégénérescence, mort neuronale, ex : Parkinson |
| Neuro-transmetteur | Neurone | Elk-1 | Cytoplasmique | Neuro-dégénérescence, ex : Parkinson |
| Mutations génétiques ou stress ou cancer ou facteurs de croissance | Cellules mitotiques | Elk-1 | nucléaire | Cancer, cycle cellulaire |

Les peptides selon la présente invention peuvent avoir la structure suivante :

| N-terminal | | | | C-terminal |
|---|---|---|---|---|
| « Séquence pénétration » | «C » | « adressage NES » | « docking domain » | |
| « Séquence pénétration » | «C » | « docking domain » | « adressage NES » | |
| « Séquence pénétration » | «C » | « adressage NES » | « S » | « docking domain » |
| « Séquence pénétration » | « S » | « docking domain » | «C » | « adressage NES » |
| « Séquence pénétration » | « S » | « docking domain » | | |
| « Séquence pénétration » | «S » | « adressage NLS » | « docking domain » | |
| « Séquence pénétration » | «S » | « docking domain » | « adressage NLS » | |
| « Séquence pénétration » | «S » | « adressage NLS » | « S » | « docking domain » |
| « Séquence pénétration » | «S » | « docking domain » | « S » | « adressage NLS » |
| « Séquence pénétration » | «C » | « adressage NLS » | « docking domain » | |
| « Séquence pénétration » | «C » | « docking domain » | « adressage NLS » | |
| « Séquence pénétration » | «C » | « adressage NLS » | « S » | « docking domain » |
| « Séquence pénétration » | «C » | « docking domain » | « S » | « adressage NLS » |
| « Séquence pénétration » | «C » | « docking domain" | « adressage NES » | |
| « Séquence pénétration » | «C » | « adressage NES » | « docking domain" | |
| « Séquence pénétration » | «C » | « docking domain" | « S » | « adressage NES » |
| « Séquence pénétration » | «C » | « adressage N ES » | « S » | « docking domain" |
| « Séquence pénétration » | «S » | « docking domain » | « adressage NLS » | |
| « Séquence pénétration » | «S » | « adressage NLS » | « docking domain » | |
| « Séquence pénétration » | «S » | « docking domain » | « S » | « adressage NLS » |
| « Séquence pénétration » | «S » | « adressage N LS » | « S » | « docking domain » |
| « Séquence pénétration » | « docking domain » | « S » | « adressage NLS » | |
| « Séquence pénétration » | «C » | « adressage NLS » | « docking domain » | |
| « Séquence pénétration » | «C » | « docking domain » | « adressage NLS » | |
| « Séquence pénétration » | «C » | « adressage NLS » | «S » | « docking domain » |
| « Séquence pénétration » | «C » | « docking domain » | «S » | « adressage NLS » |
| « docking domain » | «S » | « Séquence penetration » | | |

ou

| | | | | |
|---|---|---|---|---|
| «docking domain» | «adressage NES » | «C» | «Séquence pénétration» | |
| «adressage NES» | «docking domain» | «C» | «Séquence pénétration» | |
| «docking domain» | «S» | «adressage NES» | «C» | «Séquence pénétration» |
| «docking domain» | «S» | «Séquence pénétration» | «C» | «adressage NES» |
| «docking domain» | «S» | «Séquence pénétration» | | |
| «docking domain» | «adressage NLS» | «S» | «Séquence pénétration» | |
| «adressage NLS» | «docking domain» | «S» | «Séquence pénétration» | |
| «docking domain» | «S» | «adressage NLS» | «S» | «Séquence pénétration» |
| «adressage NLS» | «S» | «docking domain» | «S» | «Séquence pénétration» |
| «docking domain» | «adressage NLS» | «C» | «Séquence pénétration» | |
| «adressage NLS» | «docking domain» | «C» | «Séquence pénétration» | |
| «docking domain» | «S» | «adressage NLS» | «C» | «Séquence pénétration» |
| «adressage NLS» | «S» | «docking domain » | «C» | «Séquence pénétration» |
| «docking domain» | «adressage NES» | «C» | «Séquence pénétration» | |
| «adressage NES» | «docking domain» | «C» | «Séquence pénétration» | |
| «docking domain» | «S» | «adressage NES » | «C» | «Séquence pénétration» |
| «adressage NES» | « S» | «docking domain» | «C» | «Séquence pénétration» |
| «docking domain» | «adressage NLS» | «S» | «Séquence pénétration» | |
| «adressage NLS» | «docking domain» | «S» | «Séquence pénétration» | |
| «docking domain» | «S» | «adressage NLS» | «S» | «Séquence pénétration» |
| «adressage NLS» | «S» | « docking domain » | «S» | «Séquence pénétration» |
| «adressage NLS» | «S» | «docking domain» | «Séquence pénétration» | |
| «adressage NLS» | «docking domain» | «C» | «Séquence pénétration» | |
| «docking domain» | «adressage NLS» | «C» | «Séquence pénétration» | |
| «docking domain» | «S» | «adressage NLS» | «C» | «Séquence pénétration» |
| «adressage NLS» | «S» | «docking domain» | «C» | «Séquence pénétration» |
| «Séquence pentration» | «S» | «docking domain» | | |

où :
- « séquence pénétration » comprend au moins une séquence d'acides aminés permettant la pénétration dudit peptide dans une cellule ;
- « S » correspond à une séquence optionnelle de type « spacer », par exemple deux prolines, ou un acide gamma aminobutyrique, permettant une flexibilité entre la séquence pénétrante et le peptide d'ancrage ;
- « C » correspond à un site de clivage enzymatique, permettant de libérer à l'intérieur de la cellule le peptide d'ancrage et sa séquence de localisation de la séquence pénétrante ; ce site de clivage peut comprendre ou non un spacer « S », placé en Cterm, Nterm, ou de part et d'autre du site de clivage. En d'autres termes, C se réfère à C tout seul, encadré par deux S ou « flanqué » par S du côté Cter ou Nter.
- « adressage » comprend une séquence d'adressage intracellulaire de type NES ou NLS;
- « docking domain » comprend la séquence d'acides aminés d'ancrage de type FXFP ou D d'un substrat donné de ERK.

Avantageusement selon l'invention, le site de clivage permet de séparer la séquence permettant la pénétration, qui se trouve à une des extrémités du peptide, du reste du peptide. Ceci est particulièrement intéressant lorsque la séquence d'adressage est une NES : la localisation du peptide est alors encore restreinte au cytoplasme.

Les avantages des peptides selon l'invention seront mieux compris, à la lecture des exemples non limitatifs suivants.

### Exemple 1

### Peptides selon l'invention

Les peptides suivants sont synthétisés par la méthode de synthèse en phase solide.: Peptides P1, P2 et P3.

### Peptides P1 et P2 : Inhibition de l'interaction entre Elk-1 et ERK

**Peptide P1** (SEQ ID No :39)
**GRKKRRQRRR**CC*TLWQFLLHLLLD*SPAKLSFQPSGSAQVHI
dans lequel :
- Séquence permettant la pénétration: **GRKKRRQRRR** (peptide pénétrant de HIV-TAT) (SEQ ID No :1)
- Site de clivage enzymatique: CC
- Séquence d'adressage: *TLWQFLLHLLLD* (NES de Net) (SEQ ID No :18)
- Séquence « docking domain »: SPAKLSFQFPSGSAQVHI(SEQ ID No :20)

P1 pénètre dans les cellules et s'y localise dans le cytoplasme.

**Peptide P2** (SEQ ID No :40)
**GRKKRRQRRR**PPSPAKLSFQFPSGSAQVHI
dans lequel :
- Séquence permettant la pénétration : **GRKKRRQRRR** (SEQ ID No:1)
- Sequence « spacer »: PP
- Séquence « docking domain »: SPAKLSFQFPSGSAQVHI(SEQ ID No :20)
   P2 pénètre dans les cellules et adopte une localisation nucléaire.

### Peptide P3 : Inhibition de l'interaction entre MKP-3 et ERK

Peptide P3 (SEQ ID No :41)
**GRKKRRQRRR**CC*TLWQFLLHLLLD*PGIMLRRLOKGNLPVRAL (SEQ ID No :41)
dans lequel :
- Séquence permettant la pénétration : **GRKKRRQRRR** (SEQ ID No :1)
- Site de clivage enzymatique: CC
- Séquence d'adressage: *TLWQFLLHLLLD* (NES de Net) (SEQ ID No :18)
- Séquence « docking domain »: PGIMLRRLOKGNLPVRAL (SEQ ID No :36)

P3 pénètre dans les cellules et adopte une localisation cytoplasmique.

### Exemple 2

### Exemple de pénétration cellulaire et de localisation nucléaire d'un peptide selon l'invention

On utilise ici le peptide F2 selon l'invention (« peptide d'ancrage »): Il possède la même séquence que le peptide P2 (voir exemple 1), et est couplé à la FITC (fluorophore) à son extrémité C-terminale.

Il a donc la structure suivante :
[peptide pénétrant de HIV-TAT] - [spacer de type PP] - [« docking domain » de type FXFP du couple ERK/Elk-1] - [FITC]

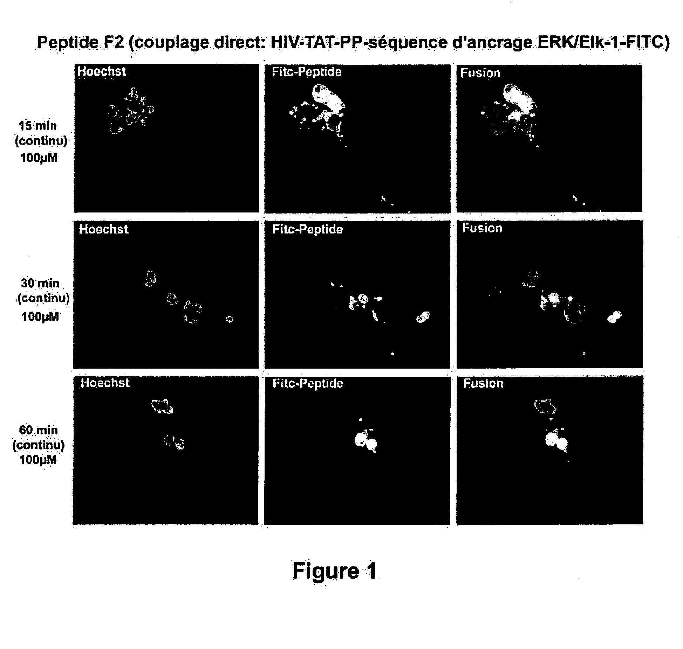

Des cellules de type HEK293 sont mises en présence du peptide F2 à différentes concentrations (1mM solution stock dans de l'eau distillée, puis dilutions à 25, 50 et 100 µM dans le milieu de culture DMEM), pendant 15, 30 ou 60 minutes, de façon continue. Les noyaux cellulaires sont marqués à l'aide de colorant Hoechst (panneaux de gauche), et le peptide F2 est visualisé grâce au marqueur FITC (panneaux du milieu). La figure 1 montre les résultats obtenus pour la concentration 100 µM en peptide F2, en fonction du temps. Les noyaux cellulaires marqués sont montrés sur les panneaux de gauche), le peptide F2 sur les panneaux du milieu et la superposition de ces deux marquages est représentée sur les panneaux de droite (panneaux notés fusion).

Le peptide F2 selon l'invention pénètre rapidement dans les cellules, et adopte une localisation nucléaire au bout de 30 minutes seulement. En l'absence de séquence d'adressage intracellulaire supplémentaire, compte tenu de la teneur riche en acides aminés basiques de la séquence de pénétration de HIV-TAT, cette dernière joue également le rôle de NLS, de sorte le peptide F2 est avantageusement localisé dans le noyau.

Le peptide F2 pénètre rapidement les cellules, puis adopte une localisation exclusivement nucléaire.

### Exemple 3

### Exemple de pénétration cellulaire et de localisation cytoplasmique d'un peptide selon l'invention

On utilise ici le peptide F1 selon l'invention (« peptide d'ancrage »): Il possède la même séquence que le peptide P1 (voir exemple 1), et est couplé à la FITC (fluorophore) à son extrémité C-terminale.

Il a donc la structure suivante :
[peptide pénétrant de HIV-TAT] - [site de clivage C-C] - [« docking domain » de type FXFP du couple ERK/Elk-1] - [FITC].

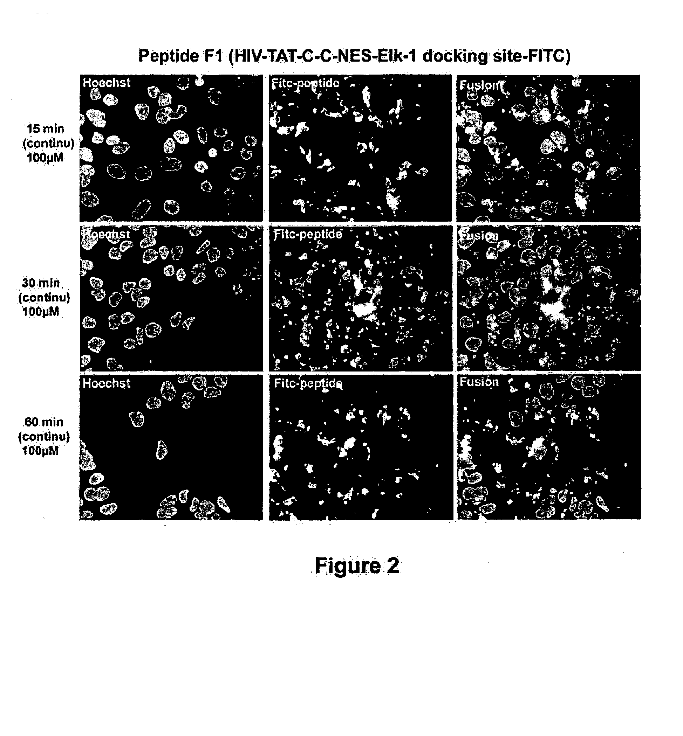

Des cellules de type HEK293 sont mises en présence du peptide F1 à différentes concentrations (1mM solution stock dans de l'eau distillée, puis dilutions à 25, 50 et 100 µM dans le milieu de culture DMEM) pendant 15, 30 ou 60 minutes, de façon continue. Les noyaux cellulaires sont marqués à l'aide de colorant Hoechst, et le peptide F1 est visualisé grâce au marqueur FITC. La figure 2 montre les résultats obtenus pour la concentration 100 µM en peptide F1, en fonction du temps.

Les noyaux cellulaires marqués sont montrés sur les panneaux de gauche), le peptide F2 sur les panneaux du milieu et la superposition de ces deux marquages est représentée sur les panneaux de droite (panneaux notés fusion).

Le peptide selon l'invention pénètre rapidement les cellules et adopte une localisation cytoplasmique.

### Exemple 4

### Caractérisation biochimique des effets inhibiteurs d'un peptide selon l'invention : P2 inhibe l'activation de Elk-1 par le sérum

On utilise ici le peptide P2 selon l'invention (voir exemple 1).

Des cellules HEK ont été traitées comme indiqué à l'exemple 2 (figure 1) par le peptide P2 (40 minutes) suivie d'un traitement par du sérum (10%) pendant 20 minutes ou 5 minutes. Le sérum active la voie MAP kinase/ERK.

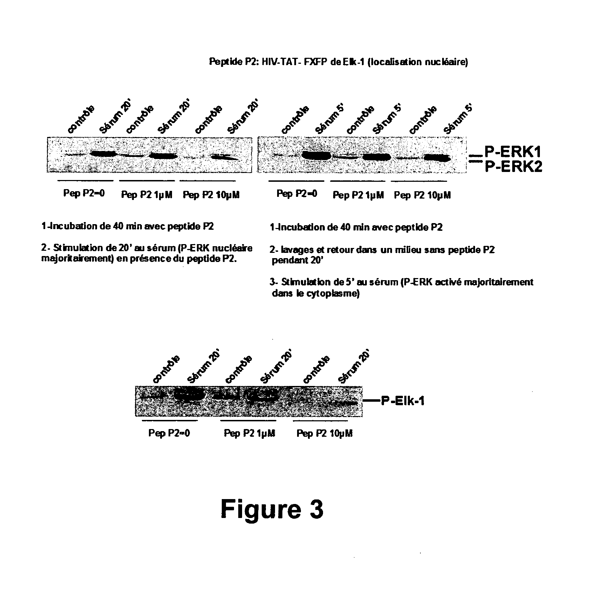

L'activation de ERK est caractérisée par western blot à l'aide d'un anticorps anti P-ERK1/2, dirigé contre la forme phosphorylée (active) de ERK (anti rabbit Phospho Thr202-Tyr204 ERK, Cell signaling, dilution 1/5000°) (figure 3, panneaux du haut). L'activation de Elk-1 est visualisée par un anticorps anti P-Elk-1 dirigé contre la forme phosphorylée de Elk-1 (anti mouse Phospho Ser383 Elk-1, Santa-Cruz, dilution 1/200°), (figure 3, panneau du bas). Des courbes dose-réponses ont été réalisées afin de déterminer la plus faible concentration de peptide efficace.

Avantageusement selon l'invention, l'induction de P-Elk-1 par le sérum est totalement inhibée en présence du peptide P2 à 10 µM. Cette inhibition est absente à 1µM de P2 (figure 3, panneau du bas). Les doses plus élevées (50 ; 100µM) de peptide P2 se montrent également efficaces sur l'inhibition de Elk-1.

Avantageusement selon l'invention, l'induction de P-ERK par le sérum n'est pas modifiée par le peptide P2 à 10µM.

### Exemple 5

### Spécificité de l'inhibition par un peptide selon l'invention :

### La phosphorylation de Elk-1 est inhibée par P2, mais pas par P1

On utilise ici les peptides P1 et P2 selon l'invention (voir exemple 1).

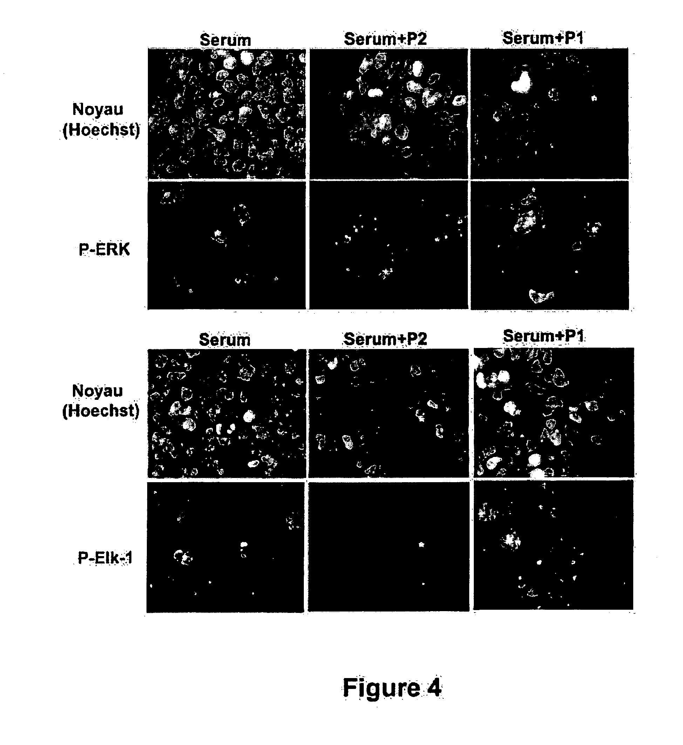

Des cellules HEK sont mises en présence du peptide P2 (figure 4, panneaux du milieu) ou P1 (figure 4, panneaux de droite), à la concentration 10 µM pendant 40 minutes. Des cellules non traitées (sans peptide) sont utilisées comme témoins (figure 4, panneaux de gauche).

Les cellules sont ensuite traitées pendant 20 minutes à l'aide de sérum de veau fétal (sérum) afin d'activer la voie MAP kinase/ERK.

La présence de la forme activée de la MAP kinase ERK est visualisée par immunodétection à l'aide d'un anticorps anti-phospho ERK (anti rabbit Phospho Thr202-Tyr204 ERK, Cell signaling, dilution 1/500° Remarque : les dilutions sont effectivement 10 fois plus faibles pour cette expérience par rapport aux western blots) et révélés par un anticorps secondaire fluorescent couplé au Cy3 (anti rabbit Cy3, Sigma, 1/2000°) (figure 4, 3 panneaux de la deuxième ligne notés P-ERK. On observe bien l'induction de P-ERK (figure 4, à titre d'exemple un marquage P-ERK est représenté par une étoile blanche, le noyau de cette cellule est signalé par une même étoile sur le panneau du haut correspondant au marquage Hoechst) quelles que soient les conditions de traitement.

La présence de la forme activée de Elk-1 est visualisée par immunocytochimie à l'aide d'un anticorps anti phospho-Ser383 de Elk-1 (anti mouse Phospho Ser383 Elk-1, Santa-Cruz, dilution 1/200°) et révélé à l'aide d'un anticorps secondaire anti mouse couplé au Cy3 (anti mouse Cy3, Jackson Immunoresearch, 1/600°) (figure 4, à titre d'exemple un marquage P-Elk-1 est représenté par une étoile blanche, sur les panneaux de la quatrième ligne gauche et droite). Les noyaux correspondant sont visualisés par la même étoile sur les panneaux de la troisième ligne notés Hoechst.

On observe l'induction de P-Elk-1 dans les compartiments cytoplasmiques et nucléaires en réponse au sérum (figure 4, panneaux de la quatrième ligne gauche), ainsi qu'en présence de sérum et du peptide P1 (figure 4, panneaux de la quatrième ligne droite).

On observe aussi l'absence d'induction de P-Elk-1 dans les cellules prétraitées avec le peptide P2 (figure 4, panneaux de la quatrième ligne au milieu).

## Revendications

1. Peptide comprenant:
- Au moins une séquence d'acides aminés permettant la pénétration dudit peptide dans une cellule ;
- Une séquence d'acides aminés d'adressage intracellulaire choisie parmi les NES ;
- Optionnellement une séquence d'adressage intracellulaire choisie parmi les NLS ;
- Une séquence d'acides aminés correspondant à une séquence d'ancrage (« docking domain ») d'un substrat d'une MAP kinase de type ERK;
- Optionnellement, au moins une séquence « spacer » ;
- Optionnellement une séquence de clivage enzymatique entourée ou non par des séquences espaceurs.

2. Peptide selon la revendication 1, tel que ladite séquence d'ancrage est choisie parmi les domaines D ou FXFP des substrats des MAP kinases de type ERK.

3. Peptide selon l'une quelconque des revendications 1 et 2, tel que ladite séquence permettant la pénétration dudit peptide dans une cellule, est choisie parmi les séquences d'un peptide pénétrant de HIV-TAT, de la Pénétratine, et les séquences 7/11R ou X7/11R.

4. Peptide selon l'une quelconque des revendications 1 à 3, couplé à un fluorophore, de préférence de manière covalente ou à une enzyme telle que la bêta-galactosidase, ou biotinylé.

5. Acide nucléique codant un peptide selon l'une quelconque des revendications 1 à 3.

6. Vecteur d'expression comprenant un acide nucléique selon la revendication 5.

7. Kit contenant au moins un peptide selon l'une quelconque des revendications 1 à 4 et/ou au moins un vecteur d'expression selon la revendication 6.

8. Utilisation d'un peptide selon l'une quelconque des revendications 1 à 4 comme inhibiteur *in vivo* ou *in vitro* de l'activité de ladite MAP kinase de type ERK envers un substrat donné dans un compartiment cellulaire donné.
